# EUROPEAN PATENT APPLICATION

(11) **EP 3 936 077 A1**
(43) Date of publication of application: **12.01.2022**
(21) Application number: 20766391.5
(22) Date of filing: 04.03.2020
(51) Int. Cl.: A61B 34/30

(54) **LIQUID PRESSURE MEDICAL DEVICE AND SURGICAL ASSISTANCE ROBOT**

(30) Priority: 06.03.2019 JP 2019040677
(71) Applicant: Kawasaki Jukogyo Kabushiki Kaisha, Hyogo 650-8670 (JP)
(72) Inventor: TANAKA, Hideki, Hyogo, 650-8670 (JP); HOMMA, Toshiyuki, Hyogo, 650-8670 (JP)
(74) Representative: Dehns
(86) International application number: PCT/JP2020/009096
(87) International publication number: WO 2020/179814

(57) **Abstract**

A hydraulic medical instrument (1A) mounted to a distal end (81) of a robotic arm includes: a linking structure (2) that is linked to the distal end (81) of the robotic arm; an insertion pipe (3) extending from the linking structure (2); and a hydraulic mover (4) provided on a distal end of the insertion pipe (3). The hydraulic medical instrument (1A) further includes a hydraulic driving source that includes at least one hydraulic pressure-dedicated shaft provided in the linking structure (2), the hydraulic driving source supplying a hydraulic liquid to the hydraulic mover (4) and collecting the hydraulic liquid from the hydraulic mover (4) by rotation or linear motion of the hydraulic pressure-dedicated shaft. When the linking structure (2) is linked to the distal end (81) of the robotic arm, the hydraulic pressure-dedicated shaft is coupled to a motor (91) disposed in the distal end (81) of the robotic arm.

## Description

### Technical Field

The present invention relates to a hydraulic medical instrument and a surgery assisting robot including the same.

### Background Art

Conventionally, surgery assisting robots to be remote-controlled by a doctor have been proposed. Some of the surgery assisting robots are, on the distal end of their robotic arm, mounted with a medical instrument such as forceps. The medical instrument that needs to be mounted differs depending on what kind of surgery is to be performed, or depending on the progress of the surgery. Therefore, the medical instrument mounted on the distal end of the robotic arm is replaced as necessary.

Conventionally, the medical instrument to be mounted on the distal end of the robotic arm is, in general, a wire-driven instrument. In recent years, hydraulic-driven medical instruments have been proposed as medical instruments mountable on the distal end of the robotic arm. For example, Patent Literature 1 discloses a hydraulic medical instrument 100, which is, as shown in FIG 14, hydraulic forceps.

The hydraulic medical instrument 100 includes: a linking structure 110 linked to the distal end of a robotic arm 150; an insertion pipe 120 extending from the linking structure 110 and inserted in the body of a patient; and a hydraulic mover 130 provided on the distal end of the insertion pipe 120.

The hydraulic mover 130 includes a piston 132, which faces a first pressure chamber 131. The linking structure 110 includes a piston 112, which faces a second pressure chamber 111. The first pressure chamber 131 and the second pressure chamber 111 are connected by a hydraulic tube 121, which extends through the inside of the insertion pipe 120.

The linking structure 110 further incorporates therein a motor 114 and a driving mechanism 113. The driving mechanism 113 drives the piston 112 by converting the rotation of the output shaft of the motor 114 into linear motion.

### Citation List

### Patent Literature

PTL 1: Japanese Laid-Open Patent Application Publication No. 2018-102633

### Summary of Invention

### Technical Problem

However, in a case where the motor is incorporated in the linking structure 110 as in the hydraulic medical instrument 100 disclosed by Patent Literature 1, the weight of the hydraulic medical instrument 100 increases. Therefore, it is desired to reduce the weight of the hydraulic medical instrument 100 to make it possible to more readily replace the hydraulic medical instrument 100.

In view of the above, an object of the present invention is to provide a hydraulic medical instrument that makes it possible to reduce the weight thereof, and to provide a surgery assisting robot including the hydraulic medical instrument.

### Solution to Problem

In order to solve the above-described problems, a hydraulic medical instrument according to the present invention is a hydraulic medical instrument mounted to a distal end of a robotic arm, the hydraulic medical instrument including: a linking structure that is linked to the distal end of the robotic arm; an insertion pipe extending from the linking structure; a hydraulic mover provided on a distal end of the insertion pipe; and a hydraulic driving source that includes at least one hydraulic pressure-dedicated shaft provided in the linking structure, the hydraulic driving source supplying a hydraulic liquid to the hydraulic mover and collecting the hydraulic liquid from the hydraulic mover by rotation or linear motion of the hydraulic pressure-dedicated shaft. When the linking structure is linked to the distal end of the robotic arm, the hydraulic pressure-dedicated shaft is coupled to a motor disposed in the distal end of the robotic arm.

According to the above configuration, the motor for moving the hydraulic pressure-dedicated shaft of the hydraulic driving source is disposed in the distal end of the robotic arm, and thereby the weight of the hydraulic medical instrument can be reduced compared to conventional hydraulic medical instruments. In addition, by merely linking the linking structure to the distal end of the robotic arm, the hydraulic pressure-dedicated shaft can be coupled to the motor.

The hydraulic pressure-dedicated shaft may rotate. The hydraulic driving source may include: a pressure chamber and a piston that faces the pressure chamber; and a driving mechanism that drives the piston by converting the rotation of the hydraulic pressure-dedicated shaft into linear motion. Among wire-driven medical instruments, there are those similar to the hydraulic medical instrument, i.e., those including a linking structure and an insertion pipe, the linking structure including therein a shaft that rotates a pulley, the shaft being coupled to a motor disposed in the distal end of a robotic arm when the linking structure is linked to the distal end of the robotic arm. Therefore, according to the above-described configuration, the hydraulic medical instrument can be replaced with a wire-driven medical instrument, and also, the motor can be used in common by the hydraulic medical instrument and the wire-driven medical instrument.

The at least one hydraulic pressure-dedicated shaft of the hydraulic driving source may include a plurality of hydraulic pressure-dedicated shafts. The driving mechanism may convert the rotation of the plurality of hydraulic pressure-dedicated shafts into linear motion. According to this configuration, the piston of the hydraulic driving source can be driven by great force.

The pressure chamber may be formed in the insertion pipe, and the piston may be disposed in the insertion pipe. This configuration makes it possible to reduce the size of the linking structure.

The hydraulic pressure-dedicated shaft may move linearly. The hydraulic driving source may include a pressure chamber and a piston that faces the pressure chamber. The hydraulic pressure-dedicated shaft may be connected to the piston. When the linking structure is linked to the distal end of the robotic arm, the hydraulic pressure-dedicated shaft may be coupled to the motor disposed in the distal end of the robotic arm via a converting mechanism that is provided in the distal end of the robotic arm, the converting mechanism converting rotation of an output shaft of the motor into linear motion. According to this configuration, the weight of the hydraulic medical instrument can be further reduced compared to a case where the hydraulic driving source includes the driving mechanism.

The insertion pipe may be configured to be bendable about at least one supporting point. The above-described hydraulic medical instrument may further include a bending mechanism that includes: a following pulley provided at the supporting point; a bending-dedicated shaft provided in the linking structure; a driving pulley fixed to the bending-dedicated shaft; and a wire wound around the following pulley and the driving pulley. When the linking structure is linked to the distal end of the robotic arm, the bending-dedicated shaft may be coupled to a motor disposed in the distal end of the robotic arm. According to this configuration, moving the hydraulic mover and bending the insertion pipe can be both performed by the motors disposed in the distal end of the robotic arm.

For example, the hydraulic mover may include a pressure chamber and a piston that faces the pressure chamber. The above-described hydraulic medical instrument may further include a hydraulic tube that extends through an inside of the insertion pipe, the hydraulic tube connecting between the pressure chamber of the hydraulic mover and the pressure chamber of the hydraulic driving source.

A surgery assisting robot according to the present invention includes: the above-described hydraulic medical instrument; and a robotic arm including a distal end to which the hydraulic medical instrument is mounted.

### Advantageous Effects of Invention

The present invention makes it possible to reduce the weight of a hydraulic medical instrument.

### Brief Description of Drawings

FIG. 1 is a perspective view of a hydraulic medical instrument and the distal end of a robotic arm according to Embodiment 1 of the present invention.
FIG. 2 is a sectional view of a linking structure of the hydraulic medical instrument and the distal end of the robotic arm shown in FIG. 1.
FIG. 3 is a sectional view of a linking configuration according to a variation.
FIG. 4 is a plan view in which the hydraulic medical instrument of FIG. 1 is shown partly in cross section.
FIG. 5 is a perspective view of the hydraulic medical instrument and the distal end of the robotic arm according to Embodiment 2 of the present invention.
FIG. 6 is a sectional view of the linking structure of the hydraulic medical instrument and the distal end of the robotic arm shown in FIG. 5.
FIG. 7 is a sectional view of a linking configuration according to a variation.
FIG. 8 is a plan view in which the hydraulic medical instrument of FIG. 5 is shown partly in cross section.
FIG. 9 is a sectional view taken along line IX-IX of FIG. 8.
FIG. 10 is a sectional view of the hydraulic medical instrument according to Embodiment 3 of the present invention.
FIG. 11 is a sectional view taken along line XI-XI of FIG. 8.
FIG. 12 is a sectional view of the linking structure according to a variation.
FIG. 13 is a sectional view taken along line XIII-XIII of FIG. 12.

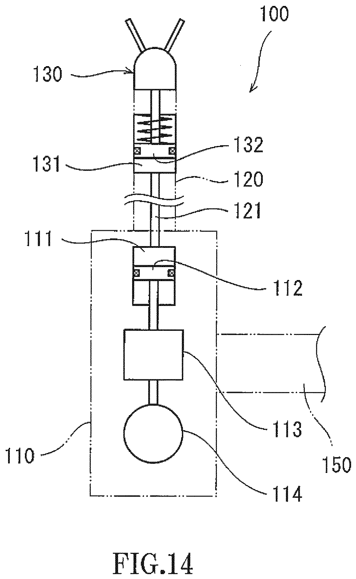

FIG. 14 shows a schematic configuration of a conventional hydraulic medical instrument.

### Description of Embodiments

### (Embodiment 1)

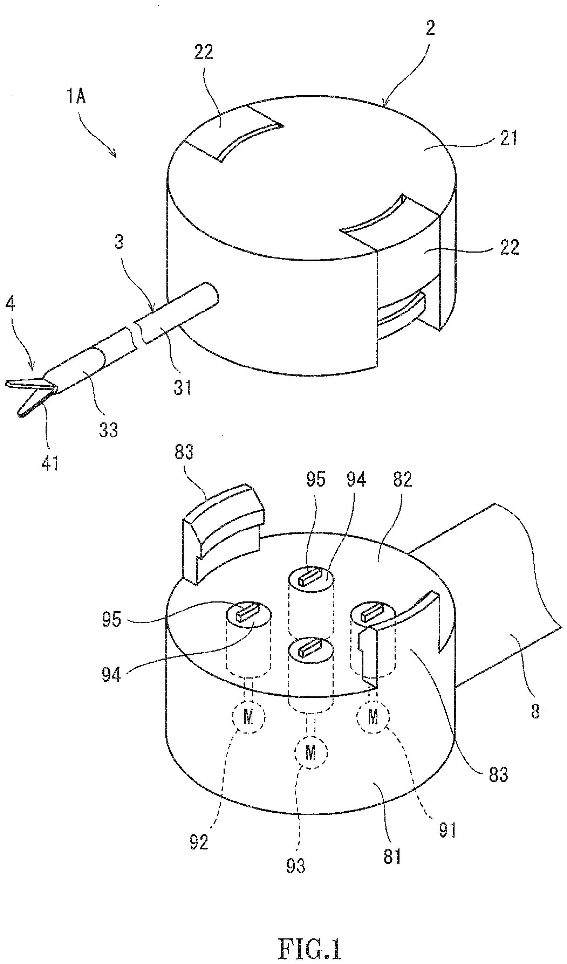

FIG. 1 shows a hydraulic medical instrument 1A according to one embodiment of the present invention. The hydraulic medical instrument 1A is mounted to a distal end 81 of a robotic arm 8. The hydraulic medical instrument 1A and the robotic arm 8 are elements of a surgery assisting robot.

The hydraulic medical instrument 1A includes: a linking structure 2 linked to the distal end of the robotic arm 8; an insertion pipe 3 extending from the linking structure 2 and inserted into the body of a patient; and a hydraulic mover 4 provided on the distal end of the insertion pipe 3. In the present embodiment, the hydraulic medical instrument 1A is hydraulic forceps, and the hydraulic mover 4 includes a gripper 41, which is opened and closed by hydraulic pressure. However, the configuration of the hydraulic mover 4 and work performed with the hydraulic mover 4 are modifiable as necessary.

In the illustrated example, the distal end 81 of the robotic arm 8 has a columnar shape, and the linking structure 2 of the hydraulic medical instrument 1A also has a columnar shape that is similar to the columnar shape of the distal end 81 of the robotic arm 8. However, the shape of the distal end 81 of the robotic arm 8 and the shape of the linking structure 2 of the hydraulic medical instrument 1A are not particularly limited.

The distal end 81 of the robotic arm 8 includes a flat linking surface 82, over which the linking structure 2 of the hydraulic medical instrument 1A is to be placed. In the present embodiment, a pair of fixing hooks 83 is provided on the peripheral edge of the linking surface 82. Hereinafter, for the sake of convenience of the description, the direction orthogonal to the linking surface 82 is referred to as the vertical direction (the direction in which the linking surface 82 faces is defined as upward, and the opposite direction is defined as downward).

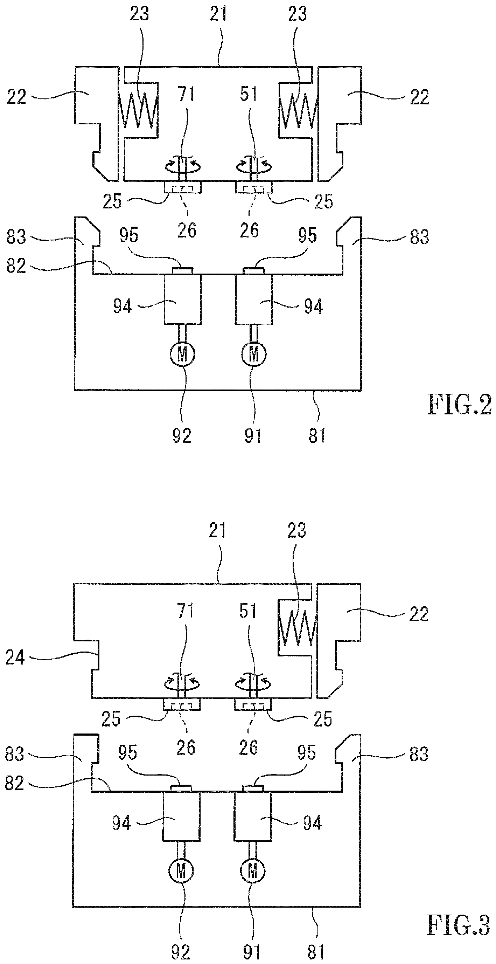

The linking structure 2 of the hydraulic medical instrument 1A includes a casing 21 and a pair of movable hooks 22. The pair of movable hooks 22 is mounted to the casing 21 at positions corresponding to the respective fixing hooks 83. The lower surface of the casing 21 faces the aforementioned linking surface 82. As shown in FIG. 2, the movable hooks 22 are urged by respective springs 23. When the linking structure 2 is inserted between the fixing hooks 83, the movable hooks 22 are automatically engaged with the fixing hooks 83, and thus the linking structure 2 is linked to the distal end 81 of the robotic arm 8.

It should be noted that the linking configuration by which the linking structure 2 and the distal end 81 of the robotic arm 8 are linked together is not limited to the one shown in FIG. 2. For example, as shown in FIG. 3, in a case where an engagement groove 24 is formed at a position, corresponding to one of the fixing hooks 83, of the casing 21, the linking structure 2 may include only one movable hook 22. Alternatively, the linking configuration by which the linking structure 2 and the distal end 81 of the robotic arm 8 are linked together may be a screw configuration.

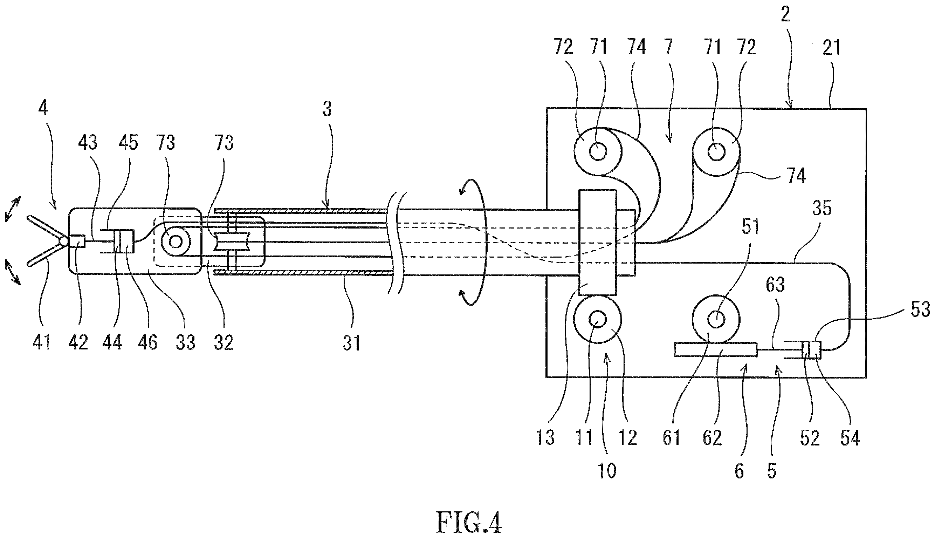

In the present embodiment, as shown in FIG. 4, the insertion pipe 3 is configured to be bendable about two supporting points. That is, the insertion pipe 3 includes a base 31, a middle portion 32, and a distal end portion 33. The base 31 is supported by the linking structure 2. The middle portion 32 is swingably coupled to the base 31. The distal end portion 33 is swingably coupled to the middle portion 32. The swinging direction of the middle portion 32 relative to the base 31, and the swinging direction of the distal end portion 33 relative to the middle portion 32, are orthogonal to each other. Alternatively, the insertion pipe 3 may be configured to be bendable about only one supporting point, or may be configured to be bendable about three or more supporting points. Further alternatively, the insertion pipe 3 may be non-bendable.

Further, in the present embodiment, the insertion pipe 3 is configured to be rollable about the center axis of the base 31. That is, the linking structure 2 supports the base 31 of the insertion pipe 3, such that the base 31 is rollable.

The hydraulic mover 4 includes, in addition to the aforementioned gripper 41, a piston 44, a rod 43, and a converting mechanism 42. The piston 44 is disposed in a cylinder 45. The rod 43 is connected to the piston 44. The converting mechanism 42 converts linear motion of the rod 43 into opening/closing motion of the gripper 41. However, the configuration of the hydraulic mover 4 is not limited to this example. As another example, the hydraulic mover 4 may include, instead of the cylinder 45 and the piston 44, a rotary actuator moved by hydraulic pressure.

The cylinder 45 may be disposed inside the distal end portion 33 of the insertion pipe 3 as a separate component, or may be configured by utilizing part of the distal end portion 33 of the insertion pipe 3. The cylinder 45 includes a tubular portion and a back wall. The back wall seals the inside of the tubular portion from the opposite side to the gripper 41. A first pressure chamber 46 is formed between the piston 44 and the back wall of the cylinder 45. That is, the piston 44 faces the first pressure chamber 46.

When a hydraulic liquid is introduced into the first pressure chamber 46, the piston 44 moves forward. When the hydraulic liquid is taken out of the first pressure chamber 46, the piston 44 moves backward. Although not illustrated, in order to assist the backward movement of the piston 44, the hydraulic mover 4 may include a spring that urges the piston 44 toward the back wall of the cylinder 45. The hydraulic liquid is not particularly limited, but may be, for example, a saline solution or an oil.

The hydraulic medical instrument 1A includes a hydraulic driving source 5, a bending mechanism 7, and a rolling mechanism 10. The hydraulic driving source 5 is intended for moving the hydraulic mover 4. The bending mechanism 7 is intended for bending the insertion pipe 3. The rolling mechanism 10 is intended for rolling the insertion pipe 3. In the present embodiment, all the components of the hydraulic driving source 5 and the rolling mechanism 10, and part of the components of the bending mechanism 7, are provided in the linking structure 2.

The hydraulic driving source 5 includes a hydraulic pressure-dedicated shaft 51, a piston 52, and a driving mechanism 6. The hydraulic pressure-dedicated shaft 51 extends in the vertical direction. The piston 52 is disposed in a cylinder 53. The driving mechanism 6 drives the piston 52 by converting rotation of the hydraulic pressure-dedicated shaft 51 into linear motion.

The cylinder 53 includes a tubular portion and a front wall. The front wall seals the inside of the tubular portion from the opposite side to the driving mechanism 6. A second pressure chamber 54 is formed between the piston 52 and the front wall of the cylinder 53. That is, the piston 52 faces the second pressure chamber 54. The second pressure chamber 54 is connected to the first pressure chamber 46 of the hydraulic mover 4 by a hydraulic tube 35, which extends through the inside of the insertion pipe 3.

With this configuration, the hydraulic driving source 5 supplies the hydraulic liquid to the hydraulic mover 4, and collects the hydraulic liquid from the hydraulic mover 4, by rotating the hydraulic pressure-dedicated shaft 51 in one direction and in the opposite direction.

In the present embodiment, the driving mechanism 6 includes a pinion 61, a rack 62, and a rod 63. The pinion 61 is fixed to the hydraulic pressure-dedicated shaft 51. The rack 62 meshes with the pinion 61. The rod 63 is connected to the rack 62 and the piston 52. However, the configuration of the driving mechanism 6 is not limited to this example, but is modifiable as necessary.

The bending mechanism 7 includes two bending-dedicated shafts 71, driving pulleys 72, following pulleys 73, and wires 74. The two bending-dedicated shafts 71 extend in the vertical direction. The driving pulleys 72 are fixed to the respective bending-dedicated shafts 71. The following pulleys 73 are provided at the aforementioned two supporting points, respectively. Each of the wires 74 is wound around a corresponding one of the following pulleys 73 and a corresponding one of the driving pulleys 72. Among these components, the bending-dedicated shafts 71 and the driving pulleys 72 are provided in the linking structure 2.

The rolling mechanism 10 includes a rolling-dedicated shaft 11, a worm (screw gear) 12, and a worm wheel (helical gear) 13. The rolling-dedicated shaft 11 extends in the vertical direction. The worm (screw gear) 12 is provided on the rolling-dedicated shaft 11. The worm wheel (helical gear) 13 is provided on the base 31 of the insertion pipe 3. Alternatively, the worm wheel may be provided on the rolling-dedicated shaft 11, and the worm may be provided on the base 31 of the insertion pipe 3. Further alternatively, the center of the rolling-dedicated shaft 11 and the center of the base 31 of the insertion pipe 3 may be made coincide with each other as seen in a plan view, and bevel gears that mesh with each other may be provided on the rolling-dedicated shaft 11 and the base 31 of the insertion pipe 3, respectively.

As shown in FIG. 2, the lower end of each of the hydraulic pressure-dedicated shaft 51, the bending-dedicated shafts 71, and the rolling-dedicated shaft 11 (not shown in FIG. 2) is connected to a corresponding one of disc-shaped couplings 25 disposed outside the casing 21 of the linking structure 2. Each coupling 25 may be integrated with the corresponding shaft. It should be noted that the couplings 25 need not be disposed outside the casing 21. The lower surface of each coupling 25 may be flush with the lower surface of the casing 21. The lower surface of each coupling 25 is provided with an engagement groove 26.

Four motors are disposed in the distal end 81 of the robotic arm 8. The four motors are a hydraulic pressure-dedicated motor 91, two bending-dedicated motors 92, and a rolling-dedicated motor 93. Each of the axial directions of the respective motors 91 to 93 is the vertical direction.

Couplings 94, each of which includes an upper surface that is flush with the aforementioned linking surface 82, are connected to the respective output shafts of the hydraulic pressure-dedicated motor 91, the bending-dedicated motors 92, and the rolling-dedicated motor 93. The upper surface of each coupling 94 is provided with a protrusion 95, which can be fitted to the engagement groove 26 of a corresponding one of the couplings 25.

Each coupling 94 is configured such that the upper surface thereof is displaceable downward by an unshown spring. That is, when the linking structure 2 of the hydraulic medical instrument 1A is linked to the distal end of the robotic arm 8, the upper surface of each coupling 94 is shifted downward by the sum of the height of its protrusion 95 and the thickness of the corresponding coupling 25 . Thereafter, as a result of the motors rotating, the orientation of the protrusions 95 and the orientation of the engagement grooves 26 coincide with each other. At the time, the unshown spring causes the upper surface of each coupling 94 to shift upward by the height of its protrusion 95. Consequently, the protrusions 95 are fitted to the respective engagement grooves 26. In this manner, each of the hydraulic pressure-dedicated shaft 51, the bending-dedicated shafts 71, and the rolling-dedicated shaft 11 is coupled to the corresponding motor via the corresponding couplings 25 and 94.

As described above, in the hydraulic medical instrument 1A of the present embodiment, the hydraulic pressure-dedicated motor 91 for moving the hydraulic pressure-dedicated shaft 51 of the hydraulic driving source 5 is disposed in the distal end 81 of the robotic arm 8, and thereby the weight of the hydraulic medical instrument 1A can be reduced compared to conventional hydraulic medical instruments. In addition, by merely linking the linking structure 2 to the distal end 81 of the robotic arm 8, the hydraulic pressure-dedicated shaft 51 can be coupled to the hydraulic pressure-dedicated motor 91.

Further, in the present embodiment, the hydraulic medical instrument 1A includes not only the hydraulic driving source 5, but also the bending mechanism 7 and the rolling mechanism 10. Therefore, moving the hydraulic mover 4, bending the insertion pipe 3, and rolling the insertion pipe 3 can be all performed by the motors disposed in the distal end 81 of the robotic arm 8.

### (Embodiment 2)

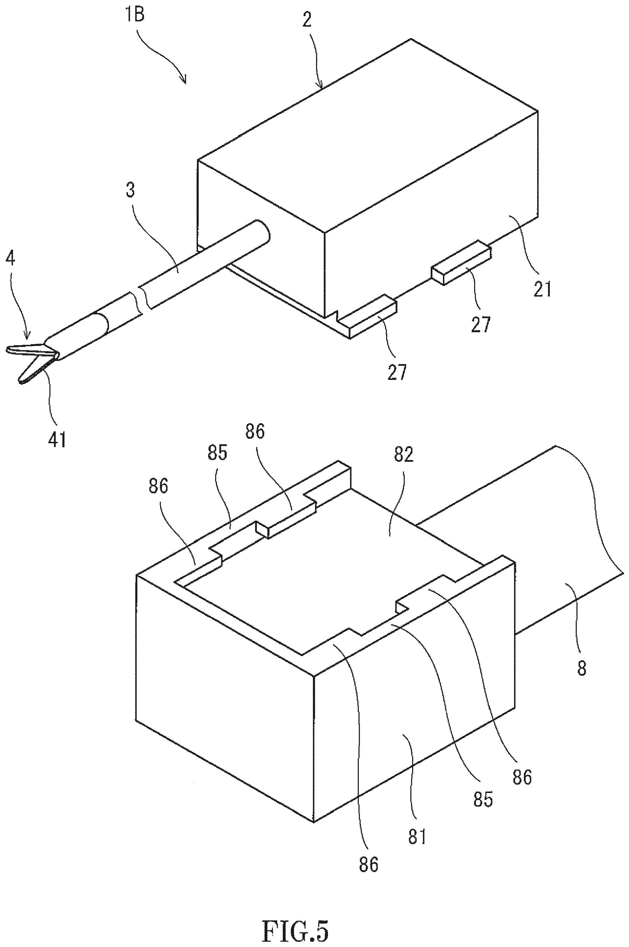

FIG. 5 shows a hydraulic medical instrument 1B according to Embodiment 2 of the present invention. It should be noted that, in the present embodiment and the following Embodiment 3, the same components as those described in Embodiment 1 are denoted by the same reference signs as those used in Embodiment 1, and repeating the same descriptions is avoided.

In the present embodiment, the distal end 81 of the robotic arm 8 and the linking structure 2 of the hydraulic medical instrument 1B are both rectangular parallelepiped and elongated in the axial direction of the insertion pipe 3. A pair of side walls 85 is provided on the linking surface 82 of the distal end 81 of the robotic arm 8 along the long sides of the linking surface 82. A pair of retaining pieces 86 protruding inward is provided on the upper end of each side wall 85. Hereinafter, for the sake of convenience of the description, the axial direction of the insertion pipe 3 is referred to as the forward-rearward direction (the direction toward the distal end side of the insertion pipe 3 is defined as forward, and the opposite direction is defined as backward).

A pair of insertion pieces 27 is provided on each side surface of the casing 21 of the linking structure 2 of the hydraulic medical instrument 1B. The linking structure 2 is placed over the linking surface 82 of the distal end 81 of the robotic arm 8, such that the insertion pieces 27 pass between the retaining pieces 86. In this state, the linking structure 2 is slid forward, and as a result, the linking structure 2 is linked to the distal end 81 of the robotic arm 8.

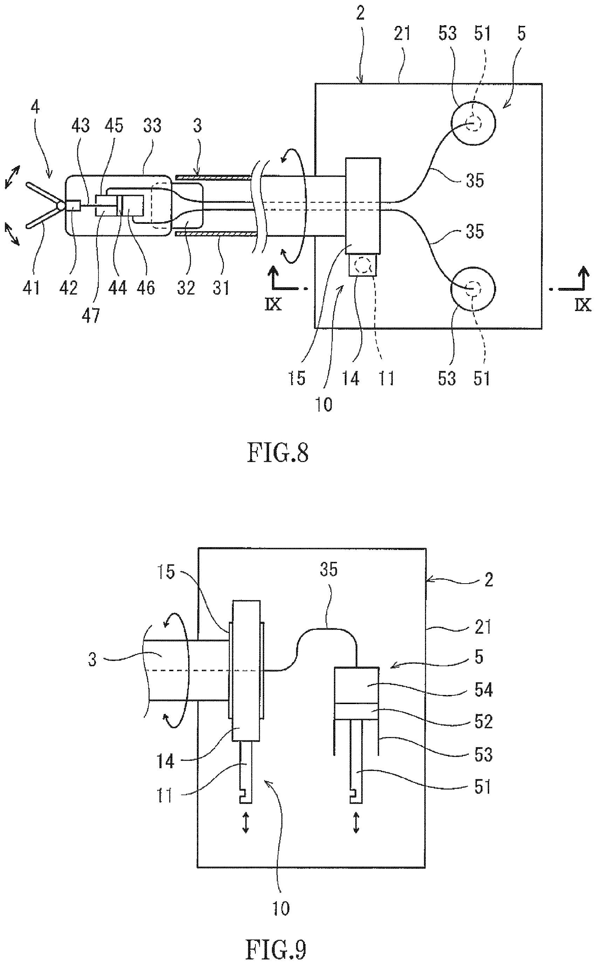

Further, in the present embodiment, as shown in FIG. 8, the cylinder 45 of the hydraulic mover 4 includes a front wall that seals the inside of the tubular portion from the gripper 41 side, and a first pressure chamber 47 is formed between the front wall of the cylinder 45 and the piston 44. That is, the piston 44 faces not only the first pressure chamber 46, but also the first pressure chamber 47.

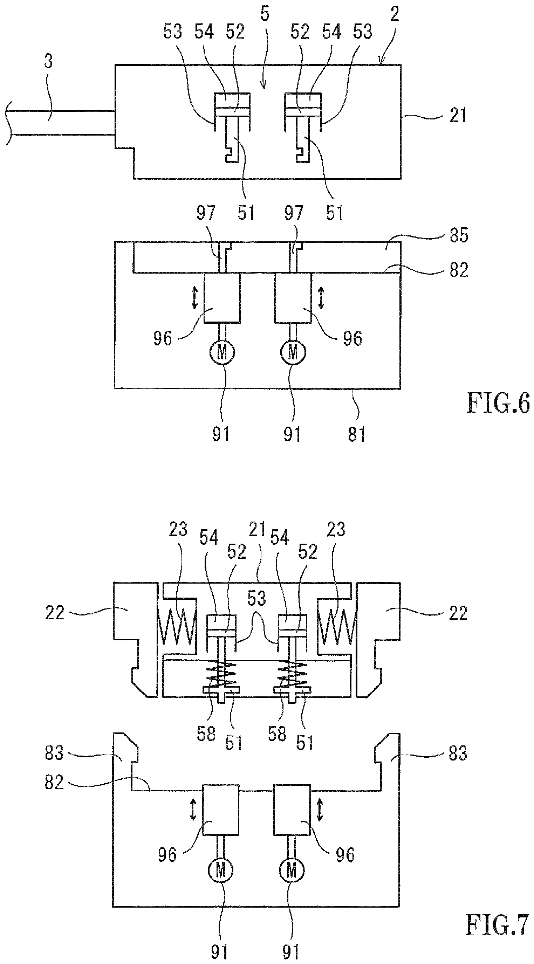

Further, in the present embodiment, as shown in FIG. 6, the hydraulic driving source 5 includes two hydraulic pressure-dedicated shafts 51 and two pistons 52. The two hydraulic pressure-dedicated shafts 51 move linearly in the vertical direction. The two pistons 52 are disposed in two cylinders 53, respectively. Two second pressure chambers 54 formed in the two respective cylinders 53 are connected to the first pressure chambers 46 and 47, respectively, by hydraulic tubes 35.

Although in FIG. 6 the hydraulic pressure-dedicated shafts 51 are arranged in the axial direction of the insertion pipe 3, FIG. 6 schematically shows the coupling configuration in which the hydraulic pressure-dedicated shafts 51 are coupled to respective hydraulic pressure-dedicated motors 91, and an actual direction in which the hydraulic pressure-dedicated shafts 51 are arranged is, as shown in FIG. 8, orthogonal to the axial direction of the insertion pipe 3. However, the direction in which the hydraulic pressure-dedicated shafts 51 are arranged is not particularly limited.

In the present embodiment, the insertion pipe 3 is configured to be bendable and rollable similar to Embodiment 1. That is, similar to Embodiment 1, the hydraulic medical instrument 1B includes the bending mechanism (the illustration of which is omitted in FIG. 8 and FIG. 9) and the rolling mechanism 10. However, the insertion pipe 3 may be non-bendable.

Further, in the present embodiment, the rolling-dedicated shaft 11 of the rolling mechanism 10 moves linearly in the vertical direction as shown in FIG. 9. The rolling-dedicated shaft 11 is provided with a rack 14. The insertion pipe 3 is provided with a pinion 15, which meshes with the rack 14.

By the linear motion of the hydraulic pressure-dedicated shafts 51, the hydraulic driving source 5 of the present embodiment supplies the hydraulic liquid to the hydraulic mover 4, and collects the hydraulic liquid from the hydraulic mover 4. To be more specific, the hydraulic driving source 5 does not include the driving mechanism 6 described in Embodiment 1, and each of the hydraulic pressure-dedicated shafts 51 is connected to a corresponding one of the pistons 52.

Further, in the present embodiment, as shown in FIG. 6, two hydraulic pressure-dedicated motors 91, two bending-dedicated motors (not shown), and one rolling-dedicated motor (not shown) are disposed in the distal end 81 of the robotic arm 8. Still further, converting mechanisms 96, which convert the rotation of the output shafts of the hydraulic pressure-dedicated motors 91 and the rotation of the output shaft of the rolling-dedicated motor into linear motion, are provided in the distal end 81 of the robotic arm 8.

Each converting mechanism 96 includes an engagement shaft 97 protruding from the linking surface 82. It should be noted that, in FIG. 1, the illustration of the engagement shaft 97 is omitted for the purpose of simplifying the drawing. The upper portion of the engagement shaft 97 is bent backward.

Meanwhile, the lower portions of the hydraulic pressure-dedicated shafts 51 and the lower portion of the rolling-dedicated shaft 11 are provided with grooves that are open forward and that can be fitted to the upper portions of the engagement shafts 97. Accordingly, by sliding the linking structure 2 forward in a state where the linking structure 2 is placed over the linking surface 82 of the distal end 81 of the robotic arm 8 as described above, the linking structure 2 is linked to the distal end 81 of the robotic arm 8, and concurrently, the hydraulic pressure-dedicated shafts 51 and the rolling-dedicated shaft 11 are coupled to the corresponding motors via the converting mechanisms 96.

In the present embodiment, similar to Embodiment 1, the hydraulic pressure-dedicated motors 91 for moving the hydraulic pressure-dedicated shafts 51 of the hydraulic driving source 5 are disposed in the distal end 81 of the robotic arm 8, and thereby the weight of the hydraulic medical instrument 1B can be reduced compared to conventional hydraulic medical instruments. In addition, by merely linking the linking structure 2 to the distal end 81 of the robotic arm 8, the hydraulic pressure-dedicated shafts 51 can be coupled to the hydraulic pressure-dedicated motors 91.

Further, in the present embodiment, since the converting mechanisms 96 are provided in the distal end 81 of the robotic arm 8, the weight of the hydraulic medical instrument 1B can be further reduced compared to a case where the hydraulic driving source 5 includes the driving mechanism 6 as in Embodiment 1.

Among wire-driven medical instruments, there are those similar to the hydraulic medical instrument 1A of Embodiment 1, i.e., those including a linking structure and an insertion pipe, the linking structure including therein shafts that rotate pulleys, the shafts being coupled to motors disposed in the distal end of a robotic arm when the linking structure is linked to the distal end of the robotic arm. Therefore, in a case where the hydraulic pressure-dedicated shaft 51 rotates as in Embodiment 1, the hydraulic medical instrument 1A can be replaced with a wire-driven medical instrument, and also, the motors can be used in common by the hydraulic medical instrument 1A and the wire-driven medical instrument.

It should be noted that, in the present embodiment, the linking configuration by which the linking structure 2 and the distal end 81 of the robotic arm 8 are linked together is not limited to the one shown in FIG. 6. For example, as shown in FIG. 7, the same fixing hooks 83 and movable hooks 22 as those of Embodiment 1 may be adopted. In this case, each of the hydraulic pressure-dedicated shafts 51 and the rolling-dedicated shaft 11 may be always pressed against the upper surface of the corresponding converting mechanism 96 by a corresponding spring 58. That is, each of the hydraulic pressure-dedicated shafts 51 and the rolling-dedicated shaft 11 is moved upward by being pushed by the corresponding converting mechanism 96, and is moved downward by the urging force of the corresponding spring 58. Thus, in a case where the hydraulic pressure-dedicated shafts 51 move linearly, the hydraulic pressure-dedicated shafts 51 can be coupled to the hydraulic pressure-dedicated motors 91 via the converting mechanisms 96 by utilizing the configuration in which the hydraulic pressure-dedicated shafts 51 and the converting mechanisms 96 are always in contact with each other.

Further, in the present embodiment, the pressure chambers are present forward and backward of the piston 44 of the hydraulic mover 4, respectively. That is, the piston 44 is a double-acting piston. Alternatively, similar to Embodiment 1, the pressure chamber may be present only backward of the piston 44. That is, the piston 44 may be a single-acting piston. In this case, the number of hydraulic pressure-dedicated shafts 51 is one. Also in Embodiment 1, similar to Embodiment 2, the number of hydraulic pressure-dedicated shafts 51 may be two, and the piston 44 may be a double-acting piston.

### (Embodiment 3)

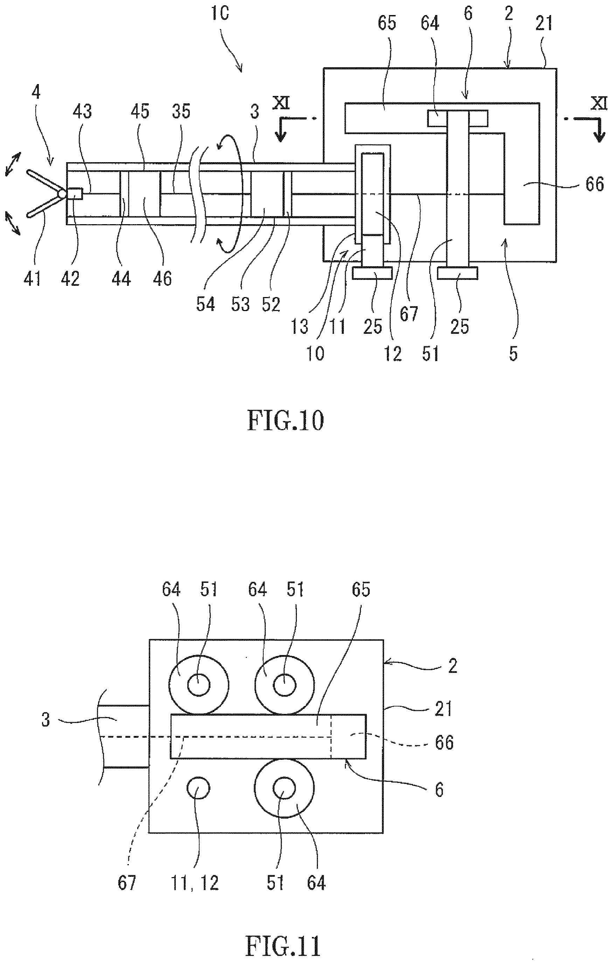

FIG. 10 shows a hydraulic medical instrument 1C according to Embodiment 3 of the present invention. In the present embodiment, since the configuration of the distal end 81 of the robotic arm 8 is the same as that described in Embodiment 1, the illustration of the distal end 81 of the robotic arm 8 is omitted.

In the present embodiment, the insertion pipe 3 is configured to be non-bendable, but rollable. Alternatively, similar to Embodiments 1 and 2, the insertion pipe 3 may be configured to be bendable.

In Embodiments 1 and 2, the second pressure chamber(s) 54 of the hydraulic driving source 5 is/are formed in the linking structure 2, and the piston(s) 52 is/are disposed in the linking structure 2. On the other hand, in the present embodiment, the second pressure chamber 54 is formed in the insertion pipe 3, and the piston 52 is disposed in the insertion pipe 3. In this case, the cylinder 53 may be disposed inside the insertion pipe 3 as a separate component, or may be configured by utilizing part of the insertion pipe 3.

Further, in the present embodiment, the hydraulic driving source 5 includes three hydraulic pressure-dedicated shafts 51, which rotate, and the driving mechanism 6 converts the rotation of the three hydraulic pressure-dedicated shafts 51 into linear motion. In other words, the driving mechanism 6 combines the rotational forces of the three hydraulic pressure-dedicated shafts 51 together, and transmits the combined force to the piston 52.

To be more specific, the driving mechanism 6 includes three pinions 64 and a rack 65. The three pinions 64 are fixed to the respective hydraulic pressure-dedicated shafts 51. The rack 65 meshes with the pinions 64. The rack 65 extends between the pinions 64 in the forward-rearward direction (the axial direction of the insertion pipe 3), and teeth are formed on both side surfaces of the rack 65. A hanging portion 66 is provided on the rear end of the rack 65, and the hanging portion 66 is connected to the piston 52 by a rod 67.

In the present embodiment, similar to Embodiment 1, hydraulic pressure-dedicated motors 91 (see FIG. 1 and FIG. 2) for moving the hydraulic pressure-dedicated shafts 51 of the hydraulic driving source 5 are disposed in the distal end 81 of the robotic arm 8, and thereby the weight of the hydraulic medical instrument 1C can be reduced compared to conventional hydraulic medical instruments. In addition, by merely linking the linking structure 2 to the distal end 81 of the robotic arm 8, the hydraulic pressure-dedicated shafts 51 can be coupled to the hydraulic pressure-dedicated motors 91.

Further, in the present embodiment, since the driving mechanism 6 of the hydraulic driving source 5 converts the rotation of the three hydraulic pressure-dedicated shafts 51 into linear motion, the piston 52 of the hydraulic driving source 5 can be driven by great force. However, the number of hydraulic pressure-dedicated shafts 51 for achieving such an advantageous effect is not limited to three, but may be two, four, or more than four.

Still further, in the present embodiment, the second pressure chamber 54 is formed in the insertion pipe 3, and the piston 52 is disposed in the insertion pipe 3. This makes it possible to reduce the size of the linking structure 2.

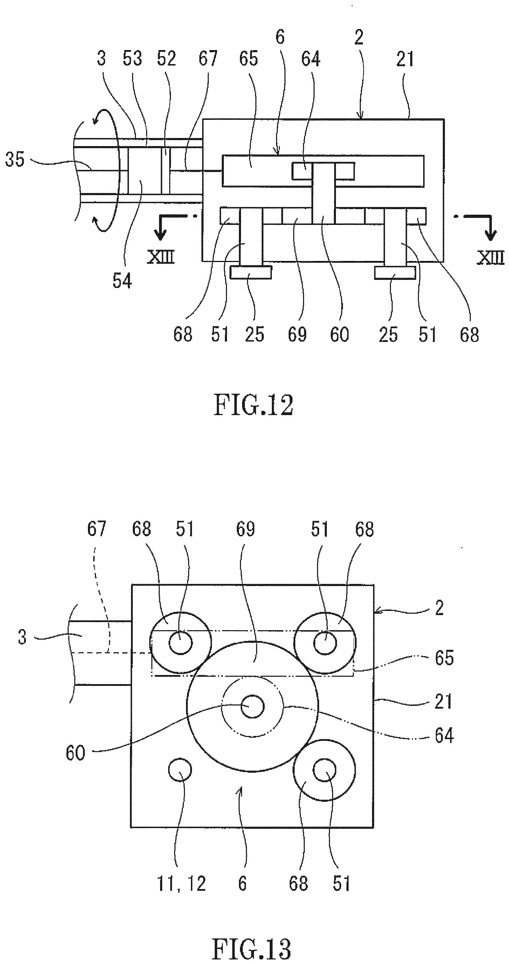

It should be noted that the configuration of the driving mechanism 6 is not limited to the one shown in FIG. 10 and FIG. 11. For example, as shown in FIG. 12 and FIG. 13, each hydraulic pressure-dedicated shaft 51 may be provided with a small gear 68, and also, a large gear 69, which meshes with all the small gears 68, may be adopted. The large gear 69 may be connected, by a middle shaft 60, to a pinion 64, which meshes with the rack 65.

### (Other Embodiments)

The present invention is not limited to the above-described embodiments. Various modifications can be made without departing from the scope of the present invention.

For example, in Embodiments 1 to 3, the insertion pipe 3 may be fixed to the linking structure 2, and the rolling mechanism 10 may be eliminated.

### Reference Signs List

- 1A to 1C: hydraulic medical instrument
- 2: linking structure
- 3: insertion pipe
- 35: hydraulic tube
- 4: hydraulic mover
- 44: piston
- 46, 47: first pressure chamber
- 5: hydraulic driving source
- 51: hydraulic pressure-dedicated shaft
- 52: piston
- 54: second pressure chamber
- 6: driving mechanism
- 7: bending mechanism
- 71: bending-dedicated shaft
- 72: driving pulley
- 73: following pulley
- 74: wire
- 8: robotic arm
- 81: distal end
- 91 to 93: motor
- 96: converting mechanism

## Claims

1. A hydraulic medical instrument mounted to a distal end of a robotic arm, the hydraulic medical instrument comprising:
a linking structure that is linked to the distal end of the robotic arm;
an insertion pipe extending from the linking structure;
a hydraulic mover provided on a distal end of the insertion pipe; and
a hydraulic driving source that includes at least one hydraulic pressure-dedicated shaft provided in the linking structure, the hydraulic driving source supplying a hydraulic liquid to the hydraulic mover and collecting the hydraulic liquid from the hydraulic mover by rotation or linear motion of the hydraulic pressure-dedicated shaft, wherein
when the linking structure is linked to the distal end of the robotic arm, the hydraulic pressure-dedicated shaft is coupled to a motor disposed in the distal end of the robotic arm.

2. The hydraulic medical instrument according to claim 1, wherein
the hydraulic pressure-dedicated shaft rotates, and
the hydraulic driving source includes:
a pressure chamber and a piston that faces the pressure chamber; and
a driving mechanism that drives the piston by converting the rotation of the hydraulic pressure-dedicated shaft into linear motion.

3. The hydraulic medical instrument according to claim 2, wherein
the at least one hydraulic pressure-dedicated shaft of the hydraulic driving source comprises a plurality of hydraulic pressure-dedicated shafts, and
the driving mechanism converts the rotation of the plurality of hydraulic pressure-dedicated shafts into linear motion.

4. The hydraulic medical instrument according to claim 2 or 3, wherein
the pressure chamber is formed in the insertion pipe, and
the piston is disposed in the insertion pipe.

5. The hydraulic medical instrument according to claim 1, wherein
the hydraulic pressure-dedicated shaft moves linearly,
the hydraulic driving source includes a pressure chamber and a piston that faces the pressure chamber,
the hydraulic pressure-dedicated shaft is connected to the piston, and
when the linking structure is linked to the distal end of the robotic arm, the hydraulic pressure-dedicated shaft is coupled to the motor disposed in the distal end of the robotic arm via a converting mechanism that is provided in the distal end of the robotic arm, the converting mechanism converting rotation of an output shaft of the motor into linear motion.

6. The hydraulic medical instrument according to any one of claims 2 to 5, wherein the insertion pipe is configured to be bendable about at least one supporting point, the hydraulic medical instrument further comprises a bending mechanism that includes:
a following pulley provided at the supporting point;
a bending-dedicated shaft provided in the linking structure;
a driving pulley fixed to the bending-dedicated shaft; and
a wire wound around the following pulley and the driving pulley, and
when the linking structure is linked to the distal end of the robotic arm, the bending-dedicated shaft is coupled to a motor disposed in the distal end of the robotic arm.

7. The hydraulic medical instrument according to any one of claims 2 to 6, wherein
the hydraulic mover includes a pressure chamber and a piston that faces the pressure chamber, and
the hydraulic medical instrument further comprises a hydraulic tube that extends through an inside of the insertion pipe, the hydraulic tube connecting between the pressure chamber of the hydraulic mover and the pressure chamber of the hydraulic driving source.

8. A surgery assisting robot comprising:
the hydraulic medical instrument according to any one of claims 1 to 7; and
a robotic arm including a distal end to which the hydraulic medical instrument is mounted.
